# EUROPEAN PATENT APPLICATION

(11) **EP 4 501 369 A1**
(43) Date of publication of application: **05.02.2025**
(21) Application number: 23788408.5
(22) Date of filing: 13.04.2023
(51) Int. Cl.: A61M 1/36

(54) **AIR TRAP CHAMBER**

(30) Priority: 13.04.2022 JP 2022066481
(71) Applicant: Nipro Corporation, Osaka 566-8510 (JP)
(72) Inventor: SAKAMOTO, Shingo, Settsu-shi, Osaka 566-8510 (JP)
(74) Representative: Germain Maureau
(86) International application number: PCT/JP2023/015091
(87) International publication number: WO 2023/199991

(57) **Abstract**

An air trap chamber comprises a housing having a tubular body part, and a cap part . The cap part has a cap ring section, a top plate section, and an inflow port. The inflow port has a liquid guide pipe. The liquid guide pipe section has a tubular shape having a vertical cavity surrounded by a liquid guide pipe side surface and a liquid guide pipe bottom surface. An outlet linked to the vertical cavity is formed in the liquid guide pipe side surface. A greatest height connecting a lowermost end and an uppermost end of an opening surface of the outlet is greater than a depth of the vertical cavity between the opening of the vertical cavity and a deeper wall at a position of an upper end of a boundary between the outlet and the vertical cavity.

## Description

### TECHNICAL FIELD

The present disclosure relates to air trap chambers.

### BACKGROUND ART

In kidney dialysis, the blood is removed from a patient having a reduced kidney function, purified, and returned to the patient. An external circulation circuit for kidney dialysis is provided with an air trap chamber for removing bubbles from the blood. The air trap chamber typically has a shape in which a cap having an inflow port is attached to the upper end of a tubular body part having an outflow port at a lower end thereof. Blood that has flowed in from the inflow port at the upper end portion flows toward the lower end due to the force of gravity, passes through a filter, and flows out from the outflow port at the lower end. A certain amount of blood is retained in the air trap chamber to form a blood layer, which allows bubbles in the blood layer to be discharged into an air layer above the blood layer.

In some air trap chambers, the blood inflow port is provided at the top plate of the cap. Such chambers facilitate handling of a blood tube, but have a problem that blood that has flowed in from the inflow port falls to the liquid surface, and therefore, foaming is likely to occur due to the impact of the falling blood. When foaming occurs, the blood is brought into contact with air and then coagulates, leading to thrombosis.

Under these circumstances, it has been studied that an outlet is formed in the side surface of a liquid guide pipe extending downward from the top plate (see, for example, PATENT DOCUMENTS 1 and 2). The formation of the outlet in the side surface of the liquid guide pipe allows the discharged blood to flow along the inside circumferential surface of the chamber as a turning flow, thereby attempting to prevent the blood from falling to the liquid surface to foam.

### CITATION LIST

### PATENT DOCUMENT

PATENT DOCUMENT 1: Japanese Unexamined Patent Publication No. 2019-193736
PATENT DOCUMENT 2: German Utility Model No. 2020-18100484

### SUMMARY OF THE INVENTION

### TECHNICAL PROBLEM

In the case in which the outlet is formed in the side surface of the liquid guide pipe, the outlet can be located at a position either higher or lower than the liquid surface of the blood layer in the chamber when the chamber is used. When the outlet is located at a position higher than the liquid surface, the shape of the outlet and the positional relationship between the outlet and the inside wall surface of the chamber are very important in order to prevent blood discharged from the outlet from falling to the liquid surface before reaching the inside wall surface of the chamber. For example, if a lower end portion of the liquid guide pipe is cut away in order to increase the size of the outlet as described in PATENT DOCUMENT 1, a portion of the discharged blood falls downward directly. For some directions and positions of the outlet, the discharged blood does not flow along the inside wall surface of the chamber.

When the outlet is located at a position lower than the liquid surface of the blood layer, blood does not fall no matter what shape the outlet has. However, unless blood is smoothly discharged to form a turning flow, the blood stagnates above the outlet. Therefore, the shape, position, and the like of the outlet are also important even when the outlet is located at a position lower than the liquid surface.

In addition, in order to form a turning flow to prevent the stagnation from occurring above the outlet, not only the shape and position of the outlet itself, but also the shapes of parts around the outlet, are also important in order to guide the flow of the discharged blood. Furthermore, the flow of blood discharged from the outlet is also affected by the shape or the like of an inner portion of the liquid guide pipe extending to the outlet.

Thus, the flow of blood discharged from the outlet is affected by various factors. These factors may have a synergistic effect, or counter effects on each other, depending on the combination thereof. Therefore, it is necessary to study various factors in order to solve problems with the flow of the discharged blood such as foaming and stagnation.

However, such study has not been sufficiently conducted for conventional air trap chambers, and various problems with the blood flow have not been overcome.

Furthermore, the liquid guide pipe is a narrow tube projecting from the top plate of the cap, and therefore, there are various constraints on the molding of the liquid guide pipe. Therefore, it is necessary to determine the configuration of the liquid guide pipe, taking moldability into account.

Still furthermore, the air trap chamber is assembled after the cap and the body part are separately molded, and therefore, it is necessary to consider the assemblability of the air trap chamber.

The present disclosure addresses the problem of solving at least one of the problems with the air trap chamber.

### SOLUTION TO THE PROBLEM

An air trap chamber according to a first embodiment of the present disclosure includes a housing having a tubular body part having an outflow port at a lower end thereof, and a cap part attached to an upper end of the body part. The cap part has a cap ring section, a top plate section closing an upper end of the cap ring section, and an inflow port provided at the top plate section. The inflow port has a liquid guide pipe section projecting downward from the top plate section. The liquid guide pipe section has a tubular shape having a vertical cavity surrounded by a liquid guide pipe side surface and a liquid guide pipe bottom surface. An outlet linked to the vertical cavity is formed in the liquid guide pipe side surface. A greatest height connecting a lowermost end and an uppermost end of an opening surface of the outlet is greater than a depth of the vertical cavity between the opening of the vertical cavity and a deeper wall at a position of an upper end of a boundary between the outlet and the vertical cavity.

In the first embodiment of the air trap chamber, the liquid guide pipe section has a tubular shape having a vertical cavity surrounded by a liquid guide pipe side surface and a liquid guide pipe bottom surface. As a result, when the outlet is located above the liquid surface of the blood layer, a liquid flowing in the liquid guide pipe section can be prevented from falling downward directly, and therefore, foaming caused by falling blood is less likely to occur. In addition, a greatest height connecting a lowermost end and an uppermost end of an opening surface of the outlet is greater than a depth of the vertical cavity between the opening of the vertical cavity and a deeper wall on the opposite side at a position of an upper end of a boundary between the outlet and the vertical cavity. Therefore, the flow of a liquid is broadened again at the outlet. Therefore, the liquid that has flowed in the vertical cavity is more likely to be drawn into the horizontal flow path, and therefore, the direction of the liquid that has flowed through the vertical cavity can be smoothly changed to the horizontal direction and then discharged from the outlet in the horizontal direction. When the outlet is located above the liquid surface of the blood layer, the discharged liquid is less likely to vertically strike the liquid surface, resulting in a reduction in foaming. When the outlet is located below the liquid surface of the blood layer, the effect of stirring the blood layer is enhanced by the liquid discharged from the opening surface, and a turning flow is formed, so that stagnation is less likely to occur in an upper portion of the blood layer. In addition, since the outlet is larger than the vertical cavity, an increase in pressure at the outlet can be reduced, and therefore, hemolysis due to a change in pressure is less likely to occur.

In the first embodiment of the air trap chamber, the vertical cavity may have a middle section at which an inside wall surface thereof closer to the outlet is an inclined surface that is inclined toward the deeper wall on the opposite side from the outlet, and an upper section above the middle section, and an inclination angle of the inclined surface may be greater than that of the upper section. With such a configuration, the liquid flowing in the vertical cavity can be concentrated toward the deeper wall to be accelerated before being discharged from the outlet, resulting in an enhancement in the effect of causing the discharged liquid to flow in the horizontal direction.

In this case, the liquid guide pipe bottom surface may extend toward the opening surface of the outlet beyond an extension of the inclined surface. With such a configuration, the accelerated liquid can be reliably directed in the horizontal direction, and therefore, the discharge liquid is less likely to fall downward.

In the first embodiment of the air trap chamber, a line connecting both side ends of the opening surface of the outlet may be tilted radially outward compared to when said line is parallel to a line connecting a central axis of the cap part and a central axis of the liquid guide pipe section. With such a configuration, the liquid discharged from the outlet can be more easily turned along the housing. The liquid discharged from the outlet might otherwise fall to the liquid surface before flowing along the housing, to foam, particularly when the outlet is located above the liquid surface. The present configuration allows the liquid to easily reach the inside wall surface of the housing, and therefore, the liquid is less likely to fall directly to the liquid surface.

In the first embodiment of the air trap chamber, a tunnel-shaped horizontal cavity may be formed between the opening surface of the outlet and the vertical cavity. With such a configuration, a strong horizontal vector can be generated for the liquid discharged from the outlet. When the outlet is located above the liquid surface, the discharged liquid is less likely to fall downward. When the outlet is located below the liquid surface, the turning flow is more likely to occur, resulting in a reduction in stagnation.

In the first embodiment of the air trap chamber, the vertical cavity may have a cylindrical upper section, and a lower section at a height position at which the outlet is formed. In a horizontal cross-section of the lower section, the vertical cavity may have a linear portion or a curved portion having a curvature smaller than that of an inside wall surface of the upper section, the linear or curved portion being lateral to the outlet. The direction of the flow moving downward in the vertical cavity is changed to the horizontal direction at the lower section. The resultant horizontal flow moves toward the outlet along the inside wall surface in the lower section. Therefore, if the portion lateral to the outlet has a great curvature, the flow is more likely to turn, resulting in a turbulent flow. By causing the curvature of the portion lateral to the outlet to be smaller than that of the upper section, in which the liquid flows downward, the horizontal flow is less likely to form a turbulent flow, and therefore, the flow can be smoothly discharged without being attenuated.

In the first embodiment of the air trap chamber, at the deeper wall on the opposite side from the outlet, an inside surface of the liquid guide pipe section at the deeper wall may be smoothly connected to the liquid guide pipe bottom surface by a curved surface. With such a configuration, the direction of the liquid flowing downward in the vertical cavity can be smoothly changed to the horizontal direction, resulting in a reduction in occurrence of stagnation in the liquid guide pipe section and the like.

In the first embodiment of the air trap chamber, a lower end surface of the liquid guide pipe section may have an ejector pin mark. In molding of the cap, mold release is performed by application of a circumferential force using a tubular pin. In the case in which a side surface through hole is provided in the liquid guide pipe, a side surface through hole formation pin and a mold release pin are likely to interfere with each other. By utilizing the lower end surface of the liquid guide pipe for mold release, the interference between the side surface through hole formation pin and the mold release pin can be reduced, and therefore, the cap including the liquid guide pipe can be precisely molded. As a result, an unintended flow due to a molding defect can be reduced in the air trap chamber.

In the first embodiment of the air trap chamber, an inside cavity of the liquid guide pipe section may be formed using a first molding pin, and a second molding pin intersecting the first molding pin. The first molding pin may be for forming an upper section of the vertical cavity. The second molding pin may be for forming a lower section of the vertical cavity and the outlet. With such a configuration, the lower section of the vertical cavity and the outlet are integrally molded using the second molding pin. As a result, the bottom surface of the liquid guide pipe is formed using the second molding pin alone, and therefore, the lower section of the vertical cavity is smoothly connected to the outlet without a step. As a result, stagnation caused by a step is less likely to occur in the lower section of the vertical cavity, and therefore, the liquid can be discharged from the outlet with a greater horizontal vector.

In the first embodiment of the air trap chamber, the body part may have a liquid surface position indicator indicating a target position of a liquid surface, and a lower end of the outlet may be located between an upper end of the body part and the liquid surface position indicator. With such a configuration, it is expected that the user is guided by the liquid surface position indicator to use the air trap chamber with the outlet located above the liquid surface of the blood layer. Even in this case, since the bottom surface of the liquid guide pipe section is closed, and the liquid is discharged from the outlet in a circumferential direction of the body part, the liquid discharged from the outlet can be prevented from falling directly to the liquid surface to foam. In particular, if the liquid surface is adjusted such that a distance between the liquid surface position indicator and the lower end of the outlet is 10-15 mm, the liquid discharged from the outlet in the circumferential direction of the body part is more likely to reach the liquid surface before being directed completely vertically, so that collision between liquids can be reduced, and therefore, foaming and thrombosis can be reduced.

In addition, the lower end of the outlet is located between the upper end of the body part and the liquid surface position indicator, so that a distance between the outlet and the top plate section can be increased, and therefore, when the liquid surface of the blood layer increases accidentally, blood is less likely to flow into other ports. Therefore, the air trap chamber can be easily used with the position of the liquid surface being higher than the position of the liquid surface indicator, and the outlet located below the liquid surface.

In the first embodiment of the air trap chamber, a stagnation reduction means may be provided between the liquid guide pipe section and an inside wall surface of the body part, and the stagnation reduction means may be a gap formed between the liquid guide pipe section and the inside wall surface of the body part, the gap becoming wider downward, or a flow rectifying wall that is formed to project from the liquid guide pipe section toward the inside wall surface of the body part at the deeper wall on the opposite side from the outlet, and is configured to guide the flow of a liquid discharged from the outlet and then flowing along the inside wall surface of the body part, inward along a radial direction of the body part.

The stagnation reduction means is provided between the liquid guide pipe section and the inside wall surface of the body part. As a result, when the outlet is adjusted to be located below the liquid surface of the blood layer, or when the liquid surface increases accidentally, so that the outlet is located below the liquid surface, stagnation is less likely to occur around the liquid guide pipe section. By providing the gap, even when the outlet is located below the liquid surface, the liquid flows and passes through the gap, so that stagnation is less likely to occur. In addition, by providing the gap, the liquid guide pipe section can be easily molded even when the liquid guide pipe section is located in the body part. If a long liquid guide pipe section is located in the body part, a situation in which the outlet is located below the liquid surface can be easily produced. By providing the flow rectifying wall, the liquid detours the liquid guide pipe section to smoothly flow, so that stagnation is less likely to occur. In particular, if a tip of the liquid guide pipe section is located inside the body part, the outlet is more likely to be located below the liquid surface, and therefore, it is preferable that the stagnation reduction means be provided.

An air trap chamber according to a second embodiment includes a housing having a tubular body part having an outflow port at a lower end thereof, and a cap part attached to an upper end of the body part. The cap part has a cap ring section, a top plate section closing an upper end of the cap ring section, and an inflow port provided at the top plate section. The inflow port has a liquid guide pipe section projecting downward from the top plate section. The liquid guide pipe section has a tubular shape having a vertical cavity surrounded by a liquid guide pipe side surface and a liquid guide pipe bottom surface. An outlet linked to the vertical cavity is formed in the liquid guide pipe side surface. The vertical cavity has a portion at which an inside wall surface thereof closer to the outlet is an inclined surface that is inclined toward a deeper wall on the opposite side from the outlet.

In the second embodiment of the air trap chamber, the liquid guide pipe section has a tubular shape having a vertical cavity surrounded by a liquid guide pipe side surface and a liquid guide pipe bottom surface, and an outlet linked to the vertical cavity is formed in the liquid guide pipe side surface. The vertical cavity has a portion at which an inside wall surface thereof closer to the outlet is an inclined surface that is inclined toward a deeper wall on the opposite side from the outlet. Therefore, the liquid flowing in the vertical cavity can be concentrated toward the deeper wall to be accelerated before being discharged from the outlet. As a result, the discharged liquid is more likely to form a turning flow, and when the outlet is located below the liquid surface of the blood layer, stagnation is less likely to occur in an upper portion of the blood layer. In addition, when the outlet is located above the liquid surface of the blood layer, the discharged liquid can be caused to flow toward the inside wall of the housing, and therefore, is less likely to strike the liquid surface vertically.

An air trap chamber according to the third embodiment includes a housing having a tubular body part having an outflow port at a lower end thereof, and a cap part attached to an upper end of the body part. The cap part has a cap ring section, a top plate section closing an upper end of the cap ring section, and an inflow port provided at the top plate section. The inflow port has a liquid guide pipe section integrally molded with the top plate section and projecting downward from the top plate section. The liquid guide pipe section has a tubular shape surrounded by a liquid guide pipe side surface and a liquid guide pipe bottom surface, and having an outlet in the liquid guide pipe side surface. A lower end surface of the liquid guide pipe section has an ejector pin mark.

In the third embodiment of the air trap chamber, in the case in which the top plate and the liquid guide pipe section are separately molded before being fixed together, a minute gap or roughness is likely to occur between the members, leading to thrombosis. If the top plate and the liquid guide pipe section are integrally molded, the risk of thrombosis can be reduced to a greater extent when the liquid surface increases. In addition, by utilizing the lower end surface of the liquid guide pipe, releasability is improved when the cap including the liquid guide pipe is molded, and therefore, the liquid guide pipe and the like can be molded more precisely. As a result, an unintended flow due to a molding defect can be reduced in the air trap chamber, and therefore, the risk of thrombosis and foaming can be reduced.

In this case, the liquid guide pipe section can be adapted to be located in the body part. Even a long liquid guide pipe section located in the body part can be easily embodied.

An air trap chamber according to a fourth embodiment includes a housing having a tubular body part having an outflow port at a lower end thereof, and a cap part attached to an upper end of the body part. The cap part has a cap ring section, a top plate section closing an upper end of the cap ring section, and an inflow port. The inflow port is fitted into the body part.

In the fourth embodiment of the air trap chamber, the cap having the inflow port is fitted into the body part. The cap having the inflow port is typically harder than the housing. Therefore, assembly precision is further improved in the case in which the cap is fitted into the housing, and therefore, the inflow port can be easily located at a predetermined position. In addition, when the cap part is attached to the body part, the cap may be held by a chunk of an automatic machine, and in this state, an adhesive may be applied to the outside circumferential surface of the cap ring section, and the cap may be directly fitted into the body part. Therefore, compared to when an adhesive agent is applied to the body part, assembly can be performed by treating only the cap part, resulting in a reduction in the number of steps in assembly.

An air trap chamber according to a fifth embodiment includes a housing having a tubular body part having an outflow port at a lower end thereof, and a cap part attached to an upper end of the body part. The cap part has a cap ring section, a top plate section closing an upper end of the cap ring section, and an inflow port provided at the top plate section. The inflow port has a liquid guide pipe section located in the body part, projecting downward from the top plate section, and having an outlet in a side surface thereof. A stagnation reduction means is provided between the liquid guide pipe section and an inside wall surface of the body part. The stagnation reduction means is a gap formed between the liquid guide pipe section and the inside wall surface of the body part, the gap becoming wider downward, or a flow rectifying wall that is formed to project from the liquid guide pipe section toward the inside wall surface of the body part at the deeper wall on the opposite side from the outlet, and is configured to guide the flow of a liquid discharged from the outlet and then flowing along the inside wall surface of the body part, inward along a radial direction of the body part.

In the fifth embodiment of the air trap chamber, the liquid guide pipe section is located in the body part, and therefore, the liquid surface can be easily adjusted such that the outlet is located below the liquid surface of the blood layer. In addition, the stagnation reduction means is provided, and therefore, even when the outlet is located below the liquid surface of the blood layer, stagnation is less likely to occur around the liquid guide pipe section. In particular, since, if a tip of the liquid guide pipe section is located inside the body part, the outlet is more likely to be located below the liquid surface, and therefore, it is preferable that the stagnation reduction means be provided.

### ADVANTAGES OF THE INVENTION

The air trap chamber of the present disclosure can solve at least one of various problems with air trap chambers.

### BRIEF DESCRIPTION OF THE DRAWINGS

[FIG. 1] FIG. 1 is a cross-sectional view illustrating an air trap chamber according to an embodiment.
[FIG. 2] FIG. 2 is an enlarged view of an upper portion of an air trap chamber.
[FIG. 3] FIG. 3 is a perspective view illustrating a cap.
[FIG. 4] FIG. 4 is a bottom view illustrating a cap.
[FIG. 5] FIG. 5 is a cross-sectional view taken along line V-V of FIG. 4.
[FIG. 6] FIG. 6 is a cross-sectional view taken along line VI-VI of FIG. 4.
[FIG. 7] FIG. 7 is a cross-sectional view illustrating an air trap chamber according to a first variation.
[FIG. 8] FIG. 8 is a bottom view illustrating a cap according to the first variation.
[FIG. 9] FIG. 9 is a perspective view illustrating a cap according to a second variation.
[FIG. 10] FIG. 10 is a cross-sectional view taken along line X-X of FIG. 9.
[FIG. 11] FIG. 11 is a cross-sectional view taken along line XI-XI of FIG. 9.
[FIG. 12] FIG. 12(a) is a cross-sectional view illustrating an upper section, and FIG. 12(b) is a cross-sectional view illustrating a lower section.
[FIG. 13] FIG. 13 is a cross-sectional view illustrating a cap according to a third variation.
[FIG. 14] FIG. 14 is a cross-sectional view illustrating the cap according to the third variation.
[FIG. 15] FIG. 15 is a front view illustrating an outlet of the cap according to the third variation.
[FIG. 16] FIG. 16 is a bottom view illustrating the cap according to the third variation.

### DESCRIPTION OF EMBODIMENTS

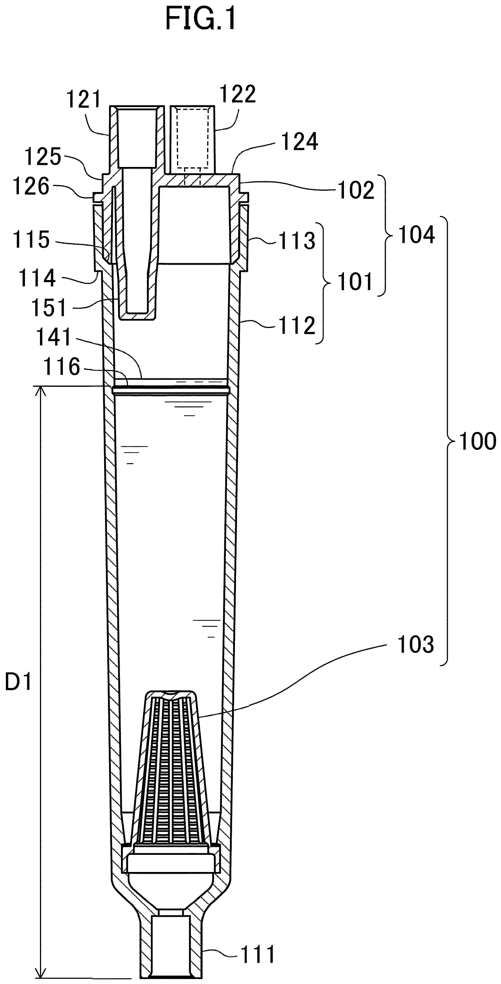

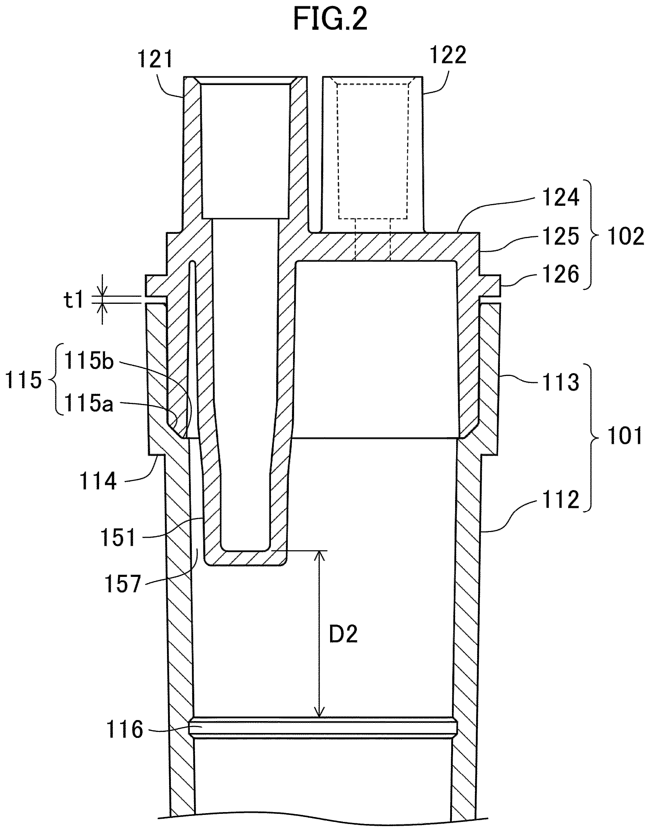

An air trap chamber according to this embodiment is, for example, used in an external circulation circuit that is used in dialysis and the like. As illustrated in FIGS. 1 and 2, the air trap chamber 100 of this embodiment has: a housing 104 having a tubular body part 101 that has an outflow port 111 at a lower end thereof, and a cap part 102 that is attached to the body part 101 so as to block an upper end thereof; and a filter 103 that is attached to a lower end portion of the body part 101. A blood inflow port 121 and other ports 122 and 123 are formed at a cap top plate section 124. The ports 122 and 123 are, for example, used to introduce a pharmaceutical liquid or replacement liquid, measure a pressure, and the like. The number of other ports is not limited to two, and may be one or three or more. The air trap chamber may have a plurality of inflow ports.

Tubes of an external circulation circuit are connected to the inflow port 121 and the outflow port 111. Blood that has flowed into a chamber container constituted by the body part 101 and the cap part 102 through the inflow port 121 forms a blood layer 141 in the chamber container, flows downward due to the force of gravity, passes through the filter 103, and flows out from the outflow port 111. During this process, bubbles occurring in the blood layer 141 are released into an air layer formed above the blood layer 141.

The body part 101 has a lower tubular section 112, and an upper tubular section 113 that is formed above the lower tubular section 112 and has a diameter greater than that of the lower tubular section 112. The filter 103 is fixed to a lower end portion of the lower tubular section 112. The lower end portion of the lower tubular section 112 below the filter 103, which has a much reduced diameter, forms the outflow port 111. On an external side of the body part 101, a body part step 114 is formed between the lower tubular section 112 and the upper tubular section 113. An inside surface flange portion 115 is formed on an internal side of the body part 101 between the lower tubular section 112 and the upper tubular section 113. The diameter of the lower tubular section 112 slightly becomes smaller downward, resulting in an improvement in releasability from a mold.

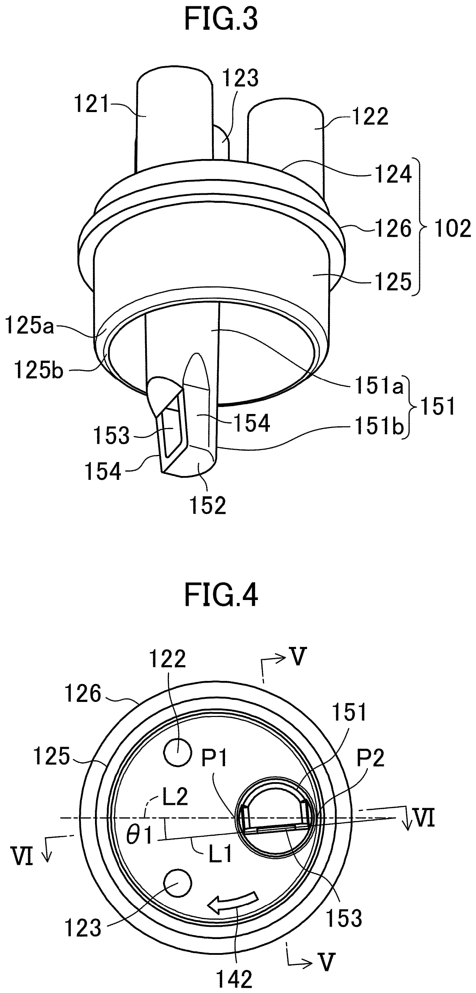

As illustrated in FIGS. 3-6, the cap part 102 has: a cap ring section 125; a cap top plate section 124 that closes an upper end of the cap ring section 125; and a cap flange section 126 that projects radially outward from the cap ring section 125. At least a portion of the cap ring section 125 that is lower than the cap flange section 126 fits into the upper tubular section 113 of the body part 101.

The inflow port 121 has a tube connection section 121a that projects upward from the cap top plate section 124, and a liquid guide pipe section 151 that projects downward from the cap top plate section 124. The liquid guide pipe section 151 projects downward beyond a lower end of the cap ring section 125. The liquid guide pipe section 151 has a tubular shape surrounded by a side surface and a bottom surface. An outlet 153 is formed in the side surface. When the cap part 102 is attached to the body part 101, the outlet 153 is located between an upper end of the body part 101 and a liquid surface position indicator 116.

Blood discharged from the outlet 153 forms a turning flow 142 that turns along an inside wall surface of the air trap chamber 100. However, the blood is bent downward due to the influence of gravity as the blood moves away from the outlet 153, and eventually flows vertically downward. In order to reduce foaming and the like caused by collision between bloods, it is preferable that the blood should reach the liquid surface before the flow direction of the blood becomes completely vertically downward. To this end, the distance between the outlet 153 and an upper surface of the blood layer 141 is preferably reduced to some extent.

A height of the liquid surface of the blood layer 141 in the air trap chamber can be set according to conditions under which the air trap chamber is used. In order to, for example, remove bubbles and reduce formation of blood foam, there is an optimum liquid surface height. For example, if the liquid surface is so low that the blood port is far away from the liquid surface, blood that has flowed out from the blood port strongly strikes the liquid surface, resulting in formation of blood foam. Therefore, a liquid surface position indicator that indicates a preferable height of the liquid surface suitable for design is provided on a side surface of the air trap chamber. The user uses the air trap chamber with the upper surface of the blood layer 141 coinciding with the liquid surface position indicator during the start of treatment.

In the air trap chamber 100 of this embodiment, the liquid surface position indicator 116, which roughly indicates the position of the upper surface of the blood layer 141, is formed on a sidewall of the lower tubular section 112. The position of the liquid surface position indicator 116 varies depending on the size, use condition, and the like of the air trap chamber. The liquid surface position indicator 116 is preferably formed above the middle of the inside of the air trap chamber 100. In order to form the blood layer 141 that is sufficient for removal of bubbles and reduce the amount of blood removed from the body, a distance D1 between the blood layer 141 and a lower end of the lower tubular section 112 may be preferably at most 12 cm, more preferably at most 10 cm. In addition, in order to reduce the situation that bubbles in the chamber flow out of the outlet port, the distance D1 is preferably at least 7 cm, more preferably at least 9 cm.

In this embodiment, a distance D2 between a lower end of the outlet 153 and the liquid surface position indicator 116, which roughly indicates the upper surface of the blood layer 141, is not particularly limited, and is preferably at most 20 mm, more preferably at most 15 mm, in order to allow blood discharged from the outlet 153 to reach the upper surface of the blood layer 141 before the flow direction of the blood becomes vertically downward. In addition, in order to produce a space between the outlet 153 and the upper surface of the blood layer 141 so as to allow the user to visually check whether blood is being discharged from the outlet 153, the distance D2 is preferably at least 5 mm, more preferably at least 10 mm.

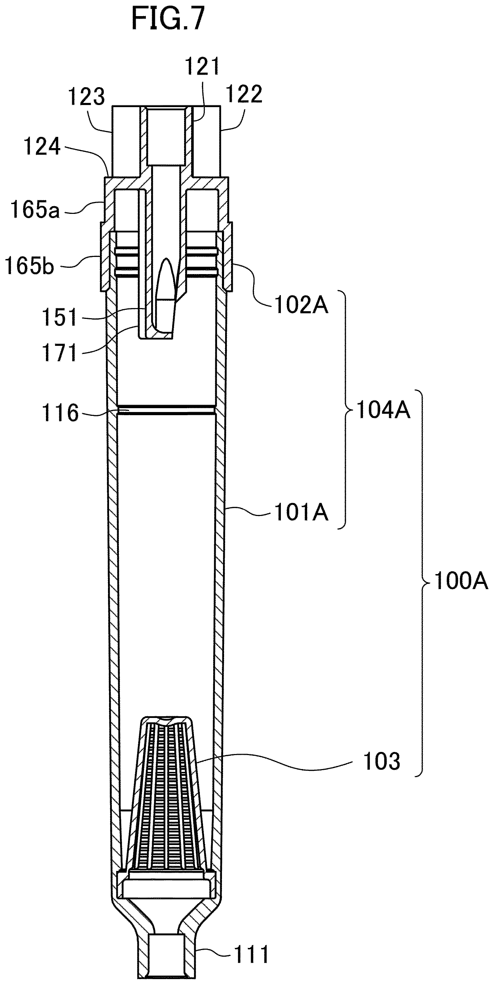

Note that the liquid surface position indicator is not limited to a linear recess as illustrated in FIG. 1 or a linear protrusion as illustrated in FIG. 7, and may have various configurations that can roughly indicate the position of the liquid surface. For example, a line can be formed by printing, graining, or the like. Alternatively, a step, a discontinuity of the tapering angle, or the like may be formed on the sidewall, and used as the liquid surface position indicator. A package insert may indicate that the liquid surface position indicator is provided, how to use the liquid surface position indicator, and the like. The liquid surface position indicator may be optionally formed, or may not be formed. Even if the liquid surface position indicator is not formed, the distance between the outlet 153 and the upper surface of the blood layer 141 may be adjusted within a predetermined range for use. In addition, an instruction manual or the like may indicate that the distance between the outlet 153 and the upper surface of the blood layer 141 should be adjusted within a predetermined range for use.

A treatment begins by adjusting the upper surface of the blood layer 141 to the position of the liquid surface indicator. However, during the treatment, the upper surface of the blood layer 141 may be shifted above the outlet 153 due to a positive pressure. When the upper surface of the blood layer 141 is located above the outlet 153, the turning flow of the blood layer 141 is obstructed by the liquid guide pipe section 151, so that blood is likely to stagnate in the vicinity of the liquid guide pipe section 151.

Therefore, in the air trap chamber 100 of this embodiment, a gap 157 that serves as a stagnation reduction means is formed between the liquid guide pipe section 151 and inside wall surfaces of the cap part 102 and the body part 101. A turning flow that is produced by blood discharged from the outlet 153 can pass through the gap 157, and therefore, is less likely to stagnate. A smallest distance between the liquid guide pipe section 151 and the inside wall surfaces of the cap part 102 and the body part 101 is preferably at least 0.5 mm, more preferably at least 1 mm. Meanwhile, if the gap 157 is too large, then when the liquid surface is located below the outlet 153 during use, blood discharged from the outlet 153 is likely to fall directly without reaching the inside wall surface. Therefore, a greatest distance between the liquid guide pipe section 151 and the inside wall surface of the body part 101 is preferably at most 3 mm, more preferably at most 2 mm.

The outlet 153 is formed to be inclined with respect to the inside wall surface such that blood discharged from the outlet 153 forms the turning flow 142 that flows along the inside wall surface. In the air trap chamber 100 of this embodiment, an opening surface of the outlet 153 is inclined with respect to a vertical cross-section extending along an imaginary line L2 connecting a position P1 on an outside circumferential surface of the liquid guide pipe section 151 closest a center of the housing 104 and a position P2 on the outside circumferential surface of the liquid guide pipe section 151 closest an inside wall surface of the housing 104 so as to approach the inside wall surface. As illustrated in FIG. 4, the imaginary line L2 connecting P1 and P2 connects a central axis of the inflow port 121 and a central axis of the cap part 102, and an imaginary line L1 indicating a direction connecting both side ends of the outlet 153 is inclined with respect to the imaginary line L2 such that the outlet 153 approaches the inside wall surface of the housing 104. As a result, the outlet 153 is tilted radially outward compared to when L1 and L2 are parallel to each other. In order to cause blood discharged from the outlet 153 to more easily form the turning flow 142 that turns along the inside wall surface of the housing 104, L1 and L2 more preferably intersect at a position outside the cap part 102 than at a central axis of the liquid guide pipe section 151, and an inclination θ1 of L1 with respect to L2 is less than 90°, specifically preferably greater than 0°, more preferably at least 3°, and preferably at most 30°, more preferably at most 20°. FIG. 4 illustrates an example in which θ1 is about 5°. Note that the turning flow 142 can be produced even if θ1 has a negative value, and therefore, θ1 can be preferably in the range of 0 to -30°, more preferably 0 to -10°.

As illustrated in FIGS. 3-6, in this embodiment, the liquid guide pipe section 151 has an upper section 151a on an upper side and a lower section 151b on a lower side. The upper section 151a is a tube having a circular cross-section. The lower section 151b has a tubular shape having two plate-shaped sidewall planes 154 opposite each other and having a non-circular cross-section that is a generally oblong circular cross-section. A middle section that is gradually deformed is formed between the upper section 151a and the lower section 151b. A flow path cross-sectional area of the lower section 151b is smaller than that of the upper section 151a. The cross-sectional area of the lower section 151b gradually becomes smaller downward. As a result, blood flowing in the liquid guide pipe section 151 is accelerated before being discharged, and therefore, the discharged blood is more easily caused to flow along the inside wall surface.

A bottom surface 152 is formed at a lower end of the lower section 151b, which is thus closed. A portion of a sidewall of the lower section 151b between the two sidewall planes 154 is cut away on one side thereof, so that the outlet 153, which is a side surface opening, is formed.

In the case in which an opening is formed in the side surface of a cylindrical tube by cutting away a portion of the cylindrical tube, the opening has an elliptical shape, and therefore, it is difficult to increase the area of the opening. In this embodiment, the lower section 151b has a tubular shape having the two flat plate-shaped sidewall planes 154 opposite each other and having a generally oblong circular cross-section, and the outlet 153 is formed by cutting away the portion of the sidewall of the lower section 151b between the two sidewall planes 154. Therefore, the outlet 153 has a rectangular shape, and therefore, an area of the opening can be easily ensured.

In addition, in this embodiment, the cut portion that is the outlet 153 reaches the bottom surface 152. Since the cut portion reaches the bottom surface 152, blood is less likely to be retained at a lower end portion of the lower section 151b. Meanwhile, if a portion of the bottom surface 152 is simply cut away, a portion of blood is likely to fall directly without striking the bottom surface 152. In this embodiment, an inside wall surface of the lower section 151b above the outlet 153 is an inclined surface 156 that has an inclination angle greater than that of the upper section 151a, and is inclined downward and toward a deeper wall on the opposite side from the outlet 153. Note that an inside wall surface of the upper section 151a may also be a vertical straight surface instead of the tapered surface. An extension of the inclined surface 156 above the outlet 153 intersects the bottom surface 152.

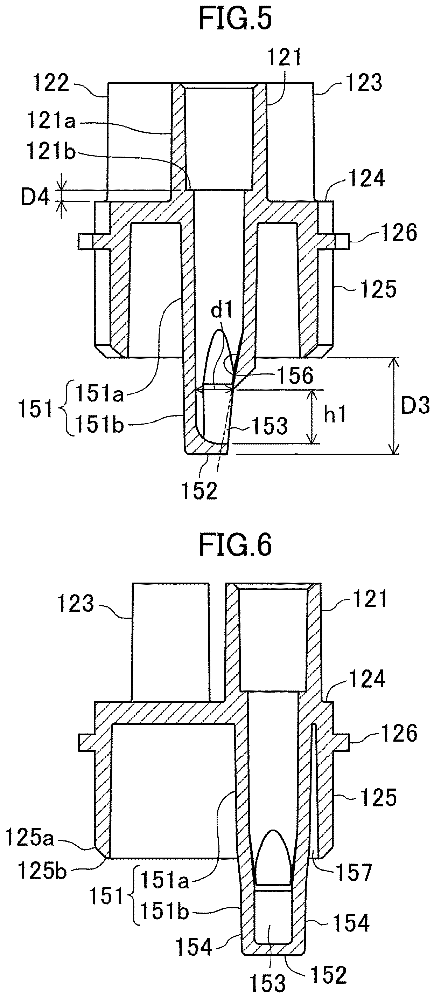

With such a configuration, blood flowing in the lower section 151b is less likely to fall downward directly. Note that FIG. 5 illustrates an example in which an end portion of the bottom surface 152 closer to the outlet 153 is located beyond an extension of the inclined surface 156 to be closer to the opening surface of the outlet 153. Alternatively, the end portion of the bottom surface 152 closer to the outlet 153 may coincide with an extension of the inclined surface 156.

In the example of FIG. 5, an inside surface of the bottom surface 152 is gently curved from the deeper wall toward the outlet 153 and inclined downward. With such a configuration, blood is lesser likely to stagnate at the lower end portion of the lower section 151b.

A projection length D3 of the liquid guide pipe section 151 from the lower end of the cap ring section 125 may be set so as to provide a suitable distance between the lower end of the outlet 153 and the upper surface of the blood layer 141. D3 is preferably at most 20 mm, more preferably at most 10 mm.

Note that the configuration of the liquid guide pipe section 151 including the outlet 153 is not limited to the preferable configuration illustrated, and may include various configurations.

In this embodiment, the cap part 102 is attached to and fitted into the upper end of the body part 101. In the case in which the cap part 102 is fitted into the upper end of the body part 101, an adhesive agent is less likely to adhere to the outlet 153 of the liquid guide pipe section 151 even in the configuration in which the liquid guide pipe section 151 is located in the body part 101. Therefore, it is less likely that the flow of blood is obstructed by a wall formed by an adhesive agent adhering to the outlet 153, so that thrombosis occurs. It is easier to form the cap part 102, which has the liquid guide pipe section 151, if the cap part 102 is formed of a material that is hard to some degree. The cap part 102 is typically harder than the body part 101. In this case, the precision of assembly can be increased when the hard cap part 102 is fitted into the body part 101 than when the hard cap part 102 is fitted onto an outer surface of the body part 101, and therefore, the liquid guide pipe section 151 can be easily arranged at a correct designed position during assembly.

Note that the embodiment in which the cap part 102 is attached to and fitted into the upper end of the body part 101 is also applied to an embodiment in which the liquid guide pipe section 151 extending downward from the cap top plate is not provided and a blood introduction port is provided at a side of the body part. The embodiment in which a cap part is attached to and fitted into the upper end of a body part allows a reduction in an external shape and an improvement in assembly efficiency. Therefore, the configuration in which a cap part is attached to and fitted into the upper end of a body part can be a means for solving the problem of reducing an external shape and improving assembly efficiency.

In this embodiment, an inclined surface 125a that is inclined toward a lower end surface 125b is formed at the lower end of the cap ring section 125. The formation of the inclined surface 125a allows the cap ring section 125 to be easily inserted into the upper tubular section 113. In addition, the inside surface flange portion 115 between the upper tubular section 113 and the lower tubular section 112 has a flange section inclined surface 115a having an inside diameter that gradually becomes smaller downward, and a flange end surface 115b extending generally horizontally from a lower end of the flange section inclined surface 115a. Therefore, the inclined surface 125a and the flange section inclined surface 115a guide the cap part 102 such that the cap part 102 is smoothly pushed into the upper tubular section 113 until the lower end surface 125b abuts the flange end surface 115b. Meanwhile, since the lower end surface 125b abuts the flange end surface 115b, the cap part 102 can be prevented from being pushed too much downward.

An inside surface of the upper tubular section 113 may be a straight surface having a uniform diameter, and an external surface of the cap ring section 125 may be a tapered surface having an outside diameter that slightly becomes smaller downward. This allows the cap ring section 125 to be easily inserted into and firmly fitted into the upper tubular section 113. Note that the inside surface of the upper tubular section 113 may have a tapered shape that slightly becomes smaller downward, and the external surface of the cap ring section 125 may have a straight surface having a uniform outside diameter.

In this embodiment, the cap part 102 has the cap flange section 126 at an upper portion of the cap ring section 125. The formation of the cap flange section 126 clearly indicates a portion to which an adhesive agent is to be applied during assembly, resulting in an improvement in ease of production. Note that in this embodiment, the cap part 102 is designed to, when fitted into the upper tubular section 113, produce a small gap t1 between a lower surface of the cap flange section 126 and an upper end surface of the body part 101. In order to allow the cap ring section 125 to be fitted into the upper tubular section 113, an outside diameter of the cap ring section 125 is slightly greater than an inside diameter of the upper tubular section 113. Therefore, when the cap ring section 125 is inserted into the upper tubular section 113, an adhesive agent applied to an outer surface of the cap ring section 125 is pushed out toward the cap flange section 126. However, even when the cap part 102 is inserted to the greatest extent possible, the gap t1 between the lower surface of the cap flange section 126 and the upper end surface of the body part 101 can prevent the adhesive agent pushed out from protruding out of the gap, so that the adhesive agent is held within the gap t1.

In this embodiment, it is, for example, recommended that the air trap chamber is used with the outlet 153 located above the liquid surface. However, the air trap chamber may be used with the outlet 153 located below the liquid surface of the blood layer 141. Even when the outlet 153 is located below the liquid surface, it is preferable that an extension of the inclined surface 156 above the outlet 153 should intersect the bottom surface 152, and a lower end of the liquid guide pipe section 151 is completely closed. With such a configuration, a portion of blood discharged from the outlet 153 that flows downward is reduced, so that a turning flow is more likely to be formed, and therefore, blood is less likely to stagnate at a portion of the blood layer 141 above the outlet 153.

In the case in which the air trap chamber is used with the outlet 153 located below the liquid surface of the blood layer 141, the liquid guide pipe section 151 is preferably sufficiently extended downward. If the liquid guide pipe section 151 is elongated, the outlet 153 can be more easily located below the liquid surface. In addition, even when the liquid surface is above the outlet 153, the distance between the liquid surface and the cap top plate section 124 can be increased, and therefore, blood is less likely to flow into other ports provided at the cap top plate section 124. In the case in which a tip of the liquid guide pipe section 151 is located inside the body part 101, the outlet 153 is likely to be located below the liquid surface, and therefore, the gap 157, or a stagnation reduction means such as a flow rectifying wall described below, is useful.

FIG. 7 illustrates an air trap chamber 100A according to a first variation. In the air trap chamber 100A, a cap part 102A is fitted onto an outer surface of a body part 101A, so that a housing 104A is formed. The body part 101A has a tapered tubular shape having a diameter that slightly becomes smaller downward. The cap part 102A has a cap lower ring section 165b having an inside diameter almost the same as an outside diameter of the body part 101A, and a cap upper ring section 165a having almost the same inside diameter as that of the body part 101A. The cap lower ring section 165b is fitted onto an outer surface of an upper end portion of the body part 101A.

In this variation, the air trap chamber 100A also has an inflow port 121 having a liquid guide pipe section 151. The liquid guide pipe section 151 projects downward beyond a lower end of the cap lower ring section 165b. In this variation, the liquid guide pipe section 151 has a flow rectifying wall 171 projecting toward an inside wall surface of a housing 104A, on a deeper wall on the opposite side from an outlet 153. The flow rectifying wall 171 serves as a stagnation reduction means with which stagnation is less likely to occur in the vicinity of the liquid guide pipe section 151.

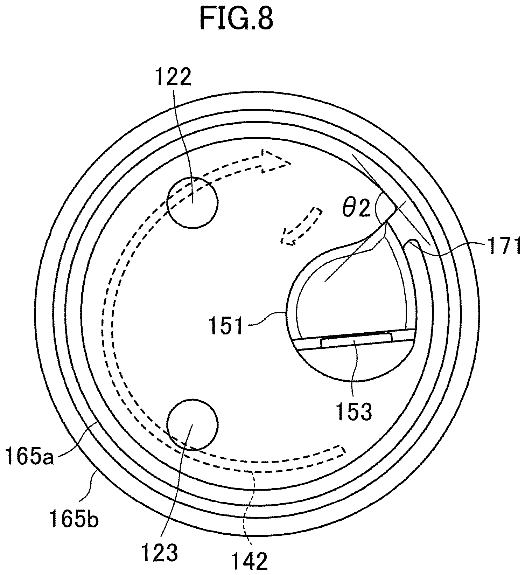

As illustrated in FIG. 8, the flow rectifying wall 171, which is inclined toward a central portion of the housing 104A, guides a turning flow 142 of blood discharged from the outlet 153 and flowing along a circular inside wall surface of the housing 104A having a uniform curvature, inward along a radial direction of the housing 104A. The flow rectifying wall 171 is formed so as to link the liquid guide pipe section 151 and an inside wall of the cap upper ring section 165a together, at a portion of the liquid guide pipe section 151 above a lower end of the cap upper ring section 165a. The flow rectifying wall 171 is also formed such that a side end surface thereof abuts an inside wall surface of the body part 101A when the body part 101A is fitted into the cap lower ring section 165b, at a portion of the liquid guide pipe section 151 below the cap upper ring section 165a. Therefore, a space between the liquid guide pipe section 151 and the inside wall surface of the housing 104A is filled by the flow rectifying wall 171 across the full length of the liquid guide pipe section 151, so that there is no gap through which blood is passed, between the liquid guide pipe section 151 and the inside wall surface of the housing 104A.

Since the space between the liquid guide pipe section 151 and the inside wall surface of the housing 104A is filled, the turning flow along the inside wall surface of the housing 104A is likely to be obstructed, which causes stagnation. However, in this variation, as illustrated in FIG. 8, an angle Θ2 between the flow rectifying wall 171 and the inside wall surface of the housing 104A (a tangential direction of the housing 104A) is increased at a portion at which the flow rectifying wall 171 is connected to the inside wall surface of the housing 104A (the cap upper ring section 165a and the body part 101A), and therefore, the turning flow can be caused to detour along the flow rectifying wall 171, so that stagnation is less likely to occur. In order to cause the turning flow to detour so that stagnation is less likely to occur, θ2 can be preferably at least 70°, more preferably at least 80°. In order to achieve moldability, θ2 is preferably at most 110°, more preferably at most 100°.

The flow rectifying wall can also be formed even in the case in which the liquid guide pipe section 151 does not extend beyond the lower end of the cap part 102 and does not project into the body part 101 or the case in which the cap inside surface and the liquid guide pipe section are integrally molded, and therefore, even when a sufficient gap is not ensured between the liquid guide pipe section and the inside wall surface of the housing, stagnation can be reduced at the liquid guide pipe section. Even in the configuration in which the cap is fitted onto the outside surface of the body part as illustrated in this variation, if a sufficient gap can be ensured between the liquid guide pipe section and the inside wall surface of the housing, a configuration in which stagnation is reduced by providing the gap can be employed. In addition, even in the case in which the cap is fitted into the body part, a configuration in which a flow rectifying wall is used to reduce stagnation can be employed.

In this variation, the air trap chamber 100A has the flow rectifying wall 171, and therefore, can have a great advantage when the outlet 153 is located below the liquid surface of the blood layer 141. However, the air trap chamber 100A can be used even when the outlet 153 is located above the liquid surface. In the case in which a tip of the liquid guide pipe section 151 is located inside the body part 101 A, a situation in which the outlet 153 is located below the liquid surface is more likely to occur, and therefore, the presence of the flow rectifying wall 171 particularly has a great effect. In addition, in this variation, the air trap chamber 100A can employ various preferable configurations illustrated for the air trap chamber 100. For example, the distance between the lower end of the outlet and the liquid surface position indicator may be adjusted, the direction of the opening surface of the outlet may be adjusted, and the shape of the liquid guide pipe section may be adjusted.

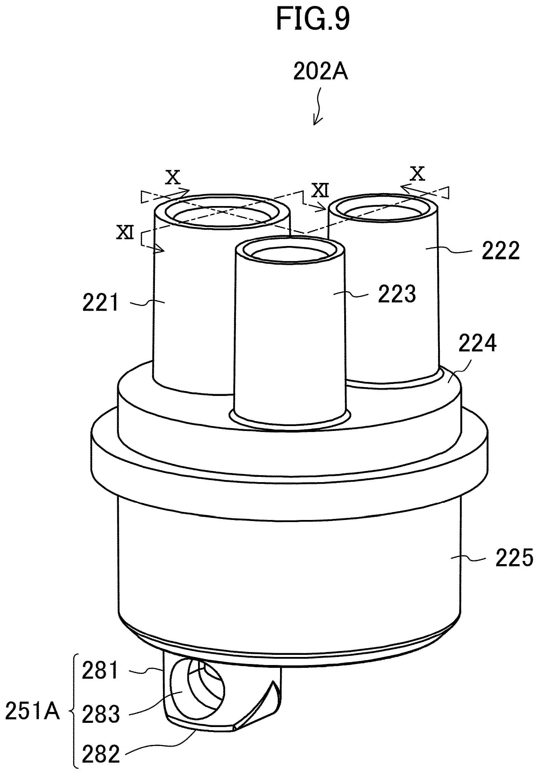

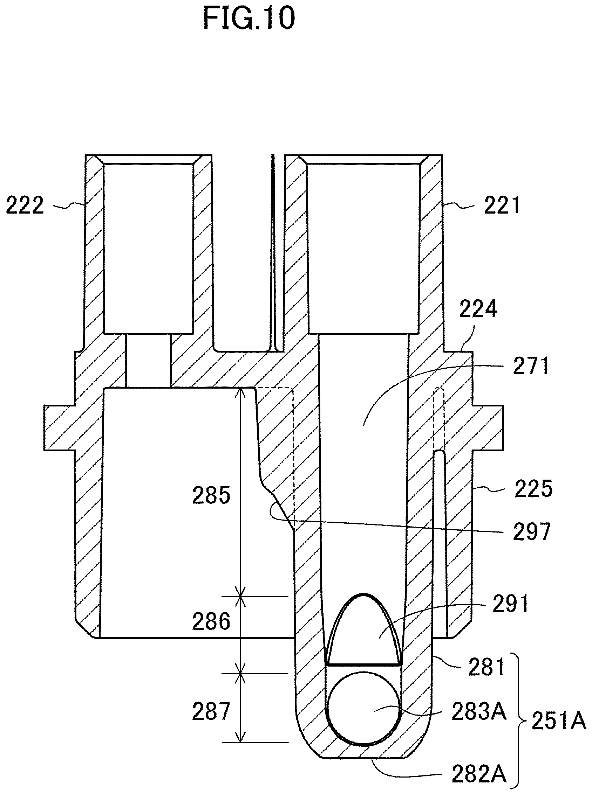

FIGS. 9-12 illustrate a cap part 202A used in an air trap chamber according to a second variation.

In the second variation, the cap part 202A has a cap ring section 225, a cap top plate section 224 that closes an upper end of the cap ring section 225, and an inflow port 221 that is provided at the cap top plate section 224. The inflow port 221 has a tubular liquid guide pipe section 251A projecting downward from the cap top plate section 224.

A lower end of the liquid guide pipe section 251A is located below a lower end of the cap ring section 225. Therefore, even when the liquid surface is located above an outlet 283A at the lower end of the liquid guide pipe section 251A, a sufficient distance can be provided between the cap top plate section 224 and the liquid surface, and therefore, blood can be prevented from flowing into port parts 222 and 223.

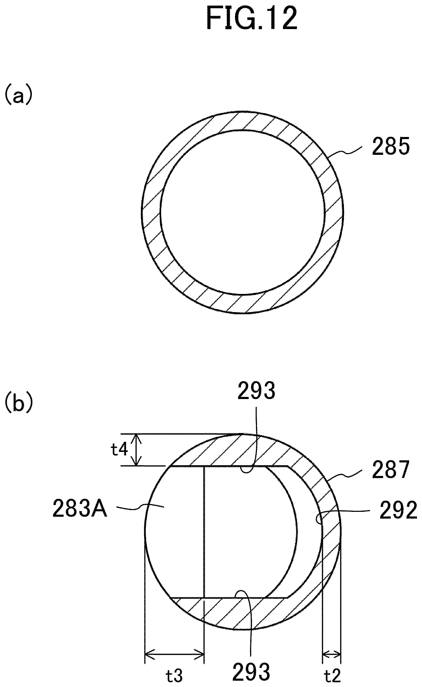

In the second variation, in the cap part 202A, the liquid guide pipe section 251A has a tubular shape having a vertical cavity 271 that is surrounded by a liquid guide pipe side surface 281, and that has a lower end closed by a liquid guide pipe bottom surface 282A. An outlet 283A is formed in the liquid guide pipe side surface 281. In this embodiment, an upper portion of the vertical cavity 271 is an upper section 285 having a perfect circular cross-section as illustrated in FIG. 12(a). A lower section 287 linked to the outlet 283A has a generally bow-shaped cross-section as illustrated in FIG. 12(b).

The outlet 283A has a tunnel-shaped horizontal cavity that has an opening surface at an outside edge of the liquid guide pipe side surface 281, and an inside edge surface at an inside edge of the liquid guide pipe side surface 281, and links an inside edge surface of the vertical cavity 271 and the opening surface. The outlet 283A has a wall not only on both lateral sides and the upper side but also on the lower side. Therefore, unlike the case in which an outlet is formed by cutting away a portion of the side surface of a liquid guide pipe including a lower end surface, a portion of blood that has flowed downward through the vertical cavity 271 can be prevented from flowing downward directly. The tunnel-shaped horizontal cavity extends from an imaginary extension of the vertical cavity to an imaginary extension of the outside edge of the liquid guide pipe side surface. In addition, a cross-sectional shape of the lower section 287 in which the outlet 283A is formed has a generally bow shape in which the outlet 283A corresponds to the chord, and a deeper wall 292 on the opposite side from the outlet 283A corresponds to the arc. The three-dimensional shape of the lower section 287 is a barrel-roof shape that is a combination of planes and a curved surface.

In the second variation, in a middle section 286 of the cap part 202A, an inside surface 291 closer to the outlet 283A gradually becomes thicker from the upper section 285 toward the lower section 287, and is inclined toward the deeper wall 292 at an angle greater than that of the upper section 285, so that the lower section 287 has a bow-shaped cross-section. Therefore, the cross-sectional area of the vertical cavity 271 of the liquid guide pipe section 251A gradually becomes smaller from the circular upper section 285 toward the bow-shaped lower section 287. As a result, blood flowing through the vertical cavity 271 is concentrated toward the deeper wall 292 in the middle section 286, and is then accelerated and redirected to reach the outlet 283A. As a result, the blood is swiftly and horizontally discharged from the outlet 283A. When the outlet 238A is located above the liquid surface of the blood layer 141, the blood discharged horizontally can be prevented from immediately falling to strike the liquid surface vertically to foam. In addition, when the outlet 238A is located below the liquid surface of the blood layer 141, the discharged blood can be caused to form a turning flow, so that the blood is less likely to stagnate. In addition, blood is less likely to stagnate in the lower section 287, where the flow direction is changed. Note that an inside surface of the upper section 285 can be either an inclined surface or a vertical straight surface.

At a lower end of the lower section 287, an inside surface of the liquid guide pipe side surface 281 and an inside surface of the liquid guide pipe bottom surface 282A are preferably connected together through a curved surface that does not have an angular bend. With such a configuration, stagnation caused by an angular portion can be avoided.

The inside surface of the liquid guide pipe bottom surface 282A may be entirely curved, instead of only a portion at which the liquid guide pipe bottom surface 282A is connected to the liquid guide pipe side surface 281. In that case, it is preferable that the inside surface of the liquid guide pipe bottom surface 282A and an inside circumferential surface of the outlet 283A should have the same curvature, and the liquid guide pipe bottom surface 282A and the outlet 283A should be registered such that a great step does not occur between the inside surface of the liquid guide pipe bottom surface 282A and the outlet 283A. With such a configuration, stagnation is lesser likely to occur at the lower section 287.

In this variation, a thickness t3 of the wall surface at the outlet 283A is greater than a thickness t2 of the deeper wall 292. In addition, a cross-section of the lower section 287 has a pair of sidewalls 293 parallel to each other between a flat surface at the outlet 283A and a curved surface at the deeper wall 292. A thickness t4 of the sidewall 293 having a flat surface can be greater than the thickness t2 of the deeper wall 292, and therefore, the strength of the liquid guide pipe section 251A is easily ensured.

The flow direction of blood that has flowed downward in the vertical cavity 271 is changed to a horizontal direction at the lower section 287, which is linked to the outlet 283A. Therefore, in the case in which the lower section 287 has a circular horizontal cross-section, and an inside wall surface of the lower section 287 is a curved surface having a uniform curvature from the deeper wall 292 to the outlet 283A, a portion having a great curvature occurs on both lateral sides of a portion linked to the outlet 283A. If there is a portion having a great curvature on both lateral sides of the portion linked to the outlet 283A, the flow whose direction has been changed to the horizontal direction flows along the inside wall surface of the lower section 287, and therefore, is likely to turn to form a turbulent flow. As illustrated in FIG. 12(b), in order to prevent a portion having a horizontal cross-section having a great curvature from occurring on both lateral sides of the portion of the lower section 287 linked to the outlet 283A, the sidewalls 293 may be flat and the lower section 287 may have a non-circular cross-section. In that case, the occurrence of the turbulent flow can be reduced at the lower section 287, and blood can be smoothly discharged from the outlet 283A without the horizontal flow being attenuated. Note that the sidewall 293 may be curved instead of being flat. In that case, the curvature of the sidewalls 293 is preferably smaller than that of the inside wall surface of the cylindrical upper section 285. In addition, only one of the sidewalls 293 on both lateral sides of the outlet may be flat or may have a shape having a curvature smaller than that of the inside wall surface of the cylindrical upper section 285. Alternatively, a wall may continue to extend to the outlet 283A with the same curvature as that of the deeper wall 292.

In this variation, the shape of an outside surface of the liquid guide pipe side surface 281 is changed according to the shape of an inside surface of the lower section 287. With such a configuration, a change in wall thickness of the lower section 287 is reduced, so that a sink mark or the like is less likely to occur during molding. Note that the shape of an outer surface of the lower section 287 may be circular as with the upper section 285.

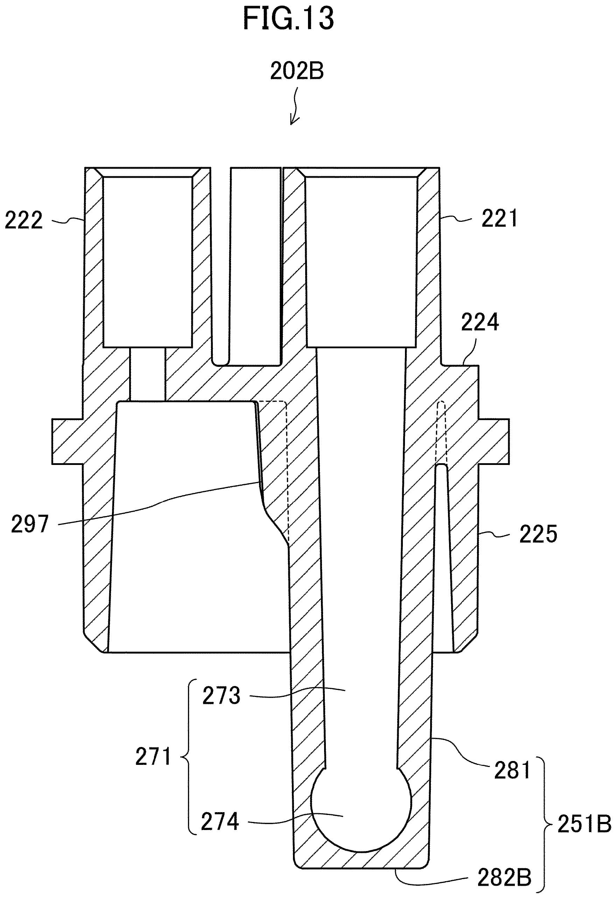

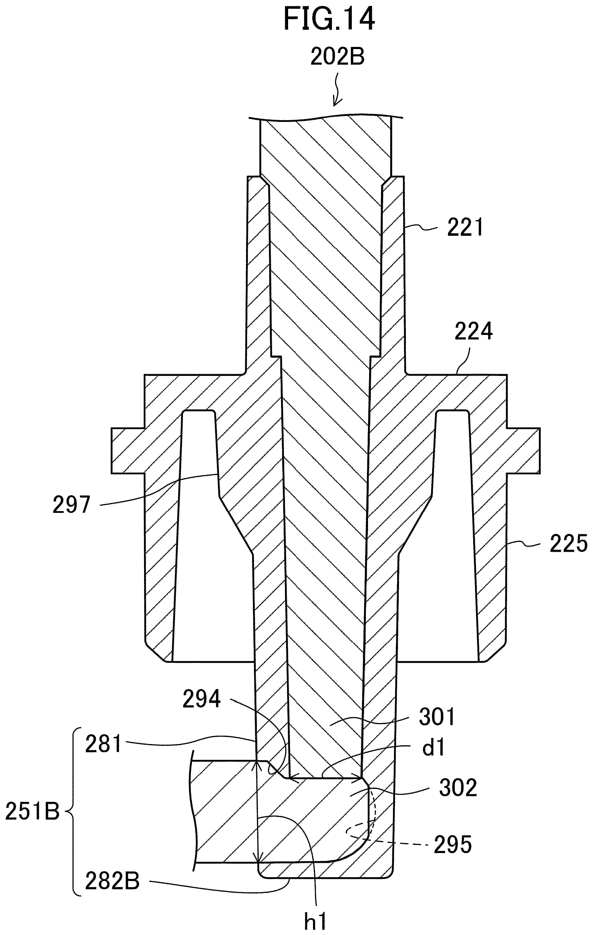

In the second variation, in the cap part 202A, a slight step occurs between the inside surface of the liquid guide pipe bottom surface 282A and the outlet 283A. Alternatively, as in a cap part 202B according to a third variation illustrated in FIGS. 13-15, an inside surface of a liquid guide pipe bottom surface 282A and a lower end portion of an outlet 283A may be connected together without a step. FIGS. 13 and 14 illustrate cross-sections at positions corresponding to line X-X and line XI-XI, respectively, in FIG. 9.

In the third variation, in the cap part 202B, a vertical cavity 271 of a liquid guide pipe section 251B has an upper section 273 that extends in the vertical direction (top-bottom direction) and has a circular cross-section, and a lower section 274 that is continuously connected to a lower end of the upper section 273 and is linked to an outlet. In this variation, the outlet 283B have different shapes at the position of an outside edge (outside surface) of a liquid guide pipe side surface 281, which is an opening surface, and the position of an inside edge (inside surface) of the liquid guide pipe side surface 281, which is a boundary between the liquid guide pipe side surface 281 and the vertical cavity 271. The outlet 283B has a circular shape at the outside edge, and a circular shape whose upper end portion is cut away, i.e., a truncated circular shape at the inside edge. A transition portion 294 at which a shape is smoothly changed is provided between the outside edge and the inside edge. Therefore, a greatest height (diameter) of the outlet 283B gradually becomes greater from the inside edge toward the outer edge of the liquid guide pipe side surface 281. The lower section 274 of the vertical cavity 271 has a cross-section having a circular shape whose upper end portion is cut away, i.e., a truncated circular shape that is the same as that at the inside edge of the outlet 283B, and extends from the outlet 283B to a deeper wall 295 on the opposite side from the outlet 283B.

The lower section of the vertical cavity and the outlet 283B are formed using the same molding pin. Therefore, the lower section 274 of the vertical cavity 271 and the outlet 283B are smoothly linked together without a step. As a result, blood is less likely to be retained in the lower section 274 of the vertical cavity 271, and can be smoothly discharged horizontally from the outlet 283B. Note that even in the third variation, the outlet 283B is a tunnel-shaped horizontal cavity that links the opening surface and the vertical cavity 271 together and is full-circumferentially surrounded as in the second variation.

In the third variation, in the cap part 202B, a greatest height h1 at an outside edge (opening surface) of the liquid guide pipe side surface 281 of the outlet 283B is greater than a depth d1 of the vertical cavity 271 at a height position of an uppermost end of a boundary between the outlet 283B and the vertical cavity 271. If the cross-sectional shape of the inside cavity at that height position is circular, the depth d1 of the vertical cavity 271 is equal to the diameter of the circle. With such a configuration, a flow that has been once constricted in the vicinity of the lower end of the upper section 273 of the vertical cavity 271 is broadened again at the opening surface of the outlet 283B. Therefore, a downward flow from the upper section 273 toward the lower section 274 of the vertical cavity 271 is more likely to be drawn in the horizontal direction in the lower section 274, and therefore, the direction of blood flowing through the vertical cavity 271 can be smoothly changed to the horizontal direction and then discharged from the outlet 283B.

In addition, since the greatest height h1 at the opening surface of the outlet 283B is greater than the depth d1 in the vicinity of the lower end of the upper section 273, an increase in pressure in the vicinity of the outlet 283B can be reduced, resulting in a decrease in pressure difference between the inside and outside of the outlet 283B. In addition, since the greatest height of the outlet 283B gradually becomes greater from the inside edge toward the outside edge of the transition portion 294, i.e., the diameter of the outlet 283B gradually becomes greater, a change in pressure can be caused to be mild when blood passes through the outlet 283B. Therefore, damage to blood cells can be reduced, and therefore, the occurrence of hemolysis can be reduced.

Note that in a forming technique described below in which a core pin abuts a slight pin, if the width of the vertical cavity 271 at the lower end of the upper section 273 is equal to the width of the boundary (the inside edge of the liquid guide pipe sidewall) between the outlet 283B and the vertical cavity 271, and is smaller than the width at the opening surface of the outlet 283B, the vertical cavity 271 and the outlet 283B can be preferably maximized.

As the ratio of h1 and d1 increases, the effect of stirring the blood layer in the chamber by blood discharged from the opening surface of the outlet 283B can be enhanced. The ratio of h1 and d1 is preferably at least 1.2, more preferably at least 1.4. Note that the size of the opening surface of the outlet 283B is limited by the size of the liquid guide pipe section 251B. Therefore, the ratio of h1 and d1 is preferably at most 1.7, more preferably at most 1.5. In addition, if the vertical cavity 271 has a tapered shape in which the diameter of the vertical cavity 271 is smallest at a height position of an uppermost end of the boundary between the outlet 283B and the vertical cavity 271, the vertical cavity 271 can temporarily smoothly constrict the flow path, resulting in a further enhancement in the effect of stirring the blood layer. Note that the vertical cavity 271 may have a uniform diameter from the position of the top plate to the height position of the upper end of the outlet 283B.

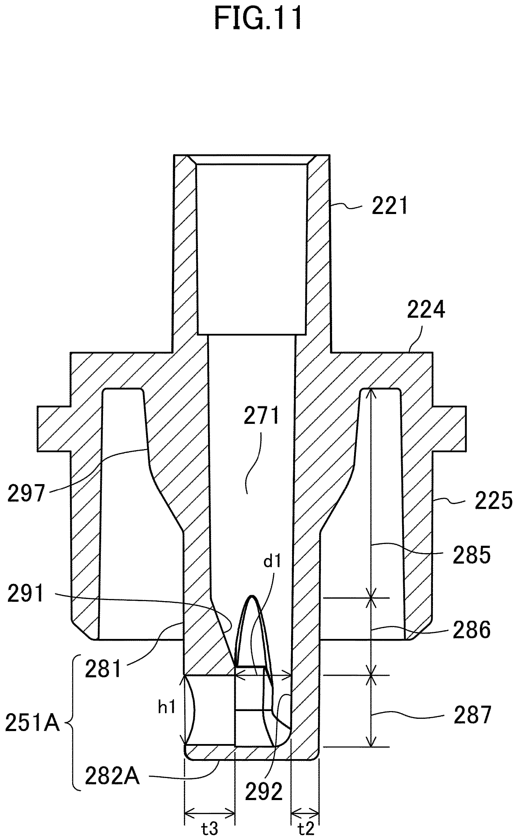

In the above configuration of the embodiment illustrated in FIG. 5, the outlet 153 consists only of the opening surface. Even in this case, if the greatest height h1 at the opening surface of the outlet 153 is greater than the depth d1 of the vertical cavity at the position of the upper end of the boundary between the outlet 153 and the vertical cavity of the liquid guide pipe section 151, a similar effect is obtained. Also in the configuration of the second variation illustrated in FIG. 11, if the greatest height h1 at the opening surface of the outlet 283A is greater than the depth d1 of the vertical cavity 271 at the position of the upper end of the boundary between the outlet 283A and the vertical cavity 271 of the liquid guide pipe section 251A, a similar effect is obtained.

In the third variation, in the cap part 202B, the deeper wall 295 on the opposite side from the outlet 283B is slightly dented, so that a step is formed. The dented portion of the deeper wall 295 preferably has a curved surface. In addition, the portion having the curved surface of the deeper wall 295 is preferably smoothly connected to an inside surface of the liquid guide pipe bottom surface 282B. With such a configuration, the direction of blood flowing downward in the liquid guide pipe section 251B can be smoothly changed to the horizontal direction at the lower end portion.

The cap part 202A of the second variation and the cap part 202B of the third variation may, for example, be formed using a mold having a core pin for forming a tubular body that is a liquid guide pipe section, and a slide pin for forming a through hole that is an outlet in the side surface of the tubular body.

The cap part 202A of the second variation may be formed using a mold havig a core pin (first pin) that reaches the liquid guide pipe bottom surface 282A of the liquid guide pipe section 251A, and a slide pin (second pin) for forming a through hole that is the outlet 283A. A portion of the core pin for forming the lower section 287 has a barrel-roof shape having a flat surface obtained by cutting away a portion of a circle. A tip surface of the slide pin for forming an outlet is caused to abut the flat surface. The core pin smoothly transitions to a circular shape, through a tapered surface, upward from a lower portion of the barrel-roof shape. The circular portion is used to form the upper section 285, and the tapered surface portion is used to form the middle section 286. If a tip of the core pin has a curved surface, the inside surface of the liquid guide pipe bottom surface 282A can be curved.

If the position of a lower end portion of the slide pin and the position of a lower end portion of the core pin are as flush as possible, a step between the outlet 283A and the inside surface of the liquid guide pipe bottom surface 282A can be reduced.

As illustrated in FIG. 14, the cap part 202B of the third variation may be formed using a mold having a core pin (first molding pin) 301 that is for forming the upper section 273 of the vertical cavity 271, and a slide pin (second molding pin) 302 that is for forming the lower section 274 of the vertical cavity 271 and the outlet 283B and that intersects the core pin. The slide pin 302 is inserted deep into the liquid guide pipe section 251A during molding. Therefore, the inside surface of the liquid guide pipe bottom surface 282B, which is a bottom surface of the lower section 274, is a smooth surface without a step formed by the slide pin 302. Therefore, stagnation is less likely to occur at the lower section 274. In the case of such a molding technique, it may be considered that in the third variation, the liquid guide pipe section 251B is configured such that the lower section 274, which is a horizontal cavity formed by the slide pin 302, is connected to the lower end of the upper section 273, which is a vertical cavity formed by the core pin 301.

A tip portion of the slide pin 302 has a barrel-roof shape whose upper end portion has a flat surface. The slide pin 302 smoothly transitions from a circular base portion to a barrel roof-shaped tip portion through a tapered surface. A flat surface of the tip portion of the slide pin 302 and a tip of the core pin 301 are caused to abut each other. The transition portion 294, which is the tapered surface of the outlet 283B, is formed by the tapered surface portion of the slide pin 302.

Since the slide pin 302 is inserted deep, a step is formed at the deeper wall 295 on the opposite side from the outlet 283B. However, if a tip of the slide pin 302 is prevented from protruding much from the position of the core pin 301, the step can be reduced. In addition, if the tip of the slide pin 302 has a curved surface, the deeper wall 295 can have a curved surface.

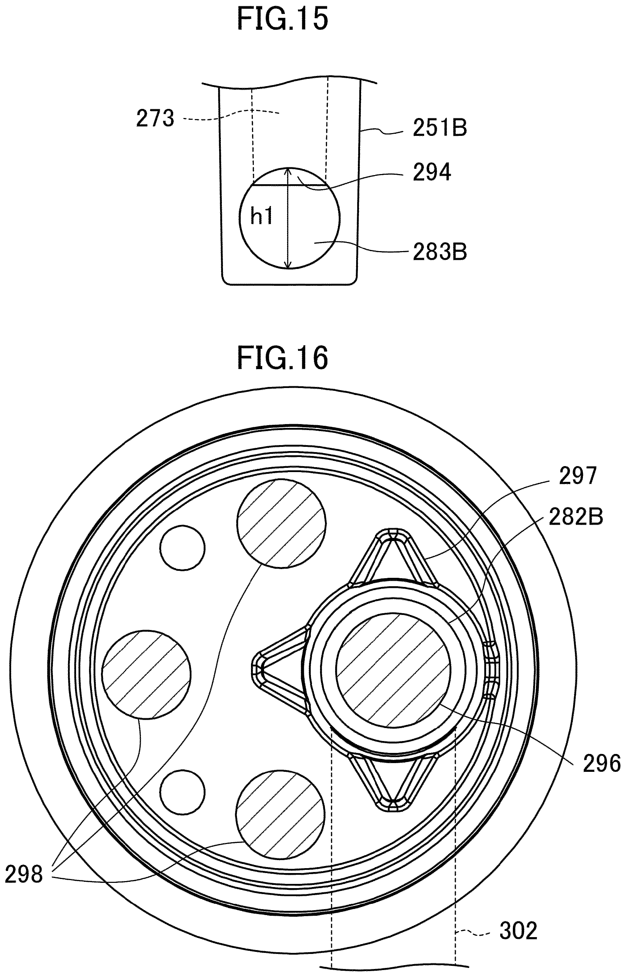

A mold for forming the cap part 202B of the third variation uses the slide pin 302. A molded article is preferably released by pressing an ejector pin against the liquid guide pipe bottom surface 282B with the core pin inside the mold and a core block externally surrounding the mold being integrated. In this case, as illustrated in FIG. 16, an ejector pin mark 296 is formed on the liquid guide pipe bottom surface 282B, which is the lower end surface of the liquid guide pipe. An ejector pin for ejecting a portion other than the lower end surface of the liquid guide pipe may be used in combination. If a plurality of ejector pins are used, force can be dispersed without being concentrated onto the lower end surface of the liquid guide pipe. In this case, an ejector pin mark 298 is formed on the lower surface of the cap top plate section 224 in addition to the lower end surface of the liquid guide pipe. In particular, as illustrated in FIG. 16, four ejector pins are preferably arranged symmetrically so that four ejector pin marks 296 and 298 are arranged symmetrically.

In the case in which the lower end surface of the liquid guide pipe is ejected using an ejector pin, a reinforcing rib 297 linking the liquid guide pipe section 251B and the cap top plate section 224 together is preferably provided. By providing the reinforcing rib 297, the strength of the liquid guide pipe section 251B can be improved, and therefore, is less likely to be deformed during ejection. Instead of the reinforcing rib 297, a reinforcing structure in which the thickness of a portion of the liquid guide pipe section 251B closer to the cap top plate section 224 is increased may be employed, for example. Note that the reinforcing structure for the liquid guide pipe such as a reinforcing rib may be optionally provided and may not be provided. Note that for the cap part 202A of the second variation, an ejector pin configuration and a liquid guide pipe reinforcing structure similar to those of the third variation may be used.

In the second and third variations, the cap top plate section 224 includes, in addition to the inflow port 221, the port parts 222 and 223, for example. The cap top plate section 224 may include, in addition to an inflow port, one or at least three ports.

In the second and third variations, the cap part can be used with the outlet located below the liquid surface. The cap part can also be used with the outlet located above the liquid surface. In the case in which the cap part is preferably used with the outlet located below the liquid surface, a liquid surface indicator that indicates the recommended position of the liquid surface may be provided on the body part, and the outlet may be located below the liquid surface indicator when the cap part is attached to the body part.

In the air trap chamber of the present disclosure, a liquid that is caused to flow from the inflow port into the chamber may be the blood. Alternatively, a replacement liquid other than the blood or other pharmaceutical liquids may be caused to flow from the inflow port into the chamber. For example, the inflow port extending from the top plate may be used as a replacement liquid introduction port, and the blood port extending laterally from the side surface of the housing may be provided below the outlet. In that case, in order to cause blood discharged from the blood port to turn along the inside surface of the housing, the blood port is preferably extended in a tangential direction of the housing, and if the flow direction of blood flowing out of the blood port and the turning direction of the inflow port are the same, a replacement liquid layer is more likely to occur in the vicinity of the liquid surface upper layer. Alternatively, the inflow port may be caused to serve as a blood introduction port, a second inflow port extending downward from the top plate section may be provided, and the second inflow port may be caused to serve as a replacement liquid introduction port. In that case, if the outlet provided lateral to the blood introduction port is located below the outlet provided lateral to the replacement liquid introduction port, and flows of the liquid flowing out from the outlets are caused to have the same turning direction, a replacement liquid layer is more likely to occur in the vicinity of the liquid surface upper layer.

The air trap chamber of the present disclosure can have embodiments described below.

An air trap chamber comprising a housing having a tubular body part having an outflow port at a lower end thereof, and a cap part attached to an upper end of the body part, wherein the body part has an inside surface flange portion, a portion of an inside wall surface of the inside surface flange portion is a flange section inclined surface having an inside diameter that becomes smaller downward, a portion of a cap ring section is fitted into an upper tubular section, a lower end of the cap ring section is an abutting surface that abuts the inside surface flange portion, and a portion of the abutting surface is an inclined surface corresponding with a flange section inclined surface.

An air trap chamber comprising a housing having a tubular body part having an outflow port at a lower end thereof, and a cap part attached to an upper end of the body part, wherein the cap part has a cap ring section, a top plate section closing an upper end of the cap ring section, and an inflow port provided at the top plate section, the inflow port has a liquid guide pipe section projecting downward from the top plate section, the liquid guide pipe section has a tubular shape having a closed bottom surface, and a side surface having an outlet for discharging a liquid in a circumferential direction of the body part, the liquid guide pipe section has a cylindrical upper section, and a tubular lower section formed below the upper section and having two flat plate-shaped sidewall planes opposite each other, and the outlet is formed at a portion interposed between the two sidewall planes of the lower section.

An air trap chamber comprising a housing having a tubular body part having an outflow port at a lower end thereof, and a cap part attached to an upper end of the body part, wherein the cap part has a cap ring section, a top plate section closing an upper end of the cap ring section, and an inflow port provided at the top plate section, the inflow port has a liquid guide pipe section projecting downward from the top plate section, the liquid guide pipe section has a tubular shape having a vertical cavity surrounded by a liquid guide pipe side surface and a liquid guide pipe bottom surface, an outlet linked to the vertical cavity is formed in the liquid guide pipe side surface, the liquid guide pipe section has a cylindrical upper section, and a tubular lower section formed below the upper section and having two flat plate-shaped sidewall planes opposite each other, a flow path cross-sectional area of the lower section is smaller than that of the upper section, an inside wall surface of the lower section above the outlet is an inclined surface that is inclined downward and toward a deeper wall on the opposite side from the outlet, and the bottom surface extends from a position of the deeper wall to at least a position at which an extension of the inside wall surface above the outlet meets a lower end of the outlet.

An air trap chamber comprising a housing having a tubular body part having an outflow port at a lower end thereof, and a cap part attached to an upper end of the body part, wherein the cap part has a cap flange section provided below a top plate section and projecting radially outward from a cap ring section, and a lower surface of the cap flange section does not abut an upper end of the body part.

An air trap chamber comprising a housing having a tubular body part having an outflow port at a lower end thereof, and a cap part attached to an upper end of the body part, wherein the cap part has a cap ring section, a top plate section closing an upper end of the cap ring section, and an inflow port provided at the top plate section, the inflow port has a liquid guide pipe section projecting downward from the top plate section, the liquid guide pipe section has a tubular shape having a vertical cavity surrounded by a liquid guide pipe side surface and a liquid guide pipe bottom surface, an outlet linked to the vertical cavity is formed in the liquid guide pipe side surface, and a tunnel-shaped horizontal cavity is formed between an opening surface of the outlet and the vertical cavity.

An air trap chamber comprising a housing having a tubular body part having an outflow port at a lower end thereof, and a cap part attached to an upper end of the body part, wherein the cap part has a cap ring section, a top plate section closing an upper end of the cap ring section, and an inflow port provided at the top plate section, the inflow port has a liquid guide pipe section projecting downward from the top plate section, the liquid guide pipe section has a tubular shape having a vertical cavity surrounded by a liquid guide pipe side surface and a liquid guide pipe bottom surface, an outlet linked to the vertical cavity is formed in the liquid guide pipe side surface, and, in a horizontal cross-section of a lower section at a height position at which the outlet is formed, the vertical cavity has a linear portion or a curved portion having a curvature smaller than that of an inside wall surface of the vertical cavity above the lower section, the linear or curved portion being lateral to the outlet.

An air trap chamber comprising a housing having a tubular body part having an outflow port at a lower end thereof, and a cap part attached to an upper end of the body part, wherein the cap part has a cap ring section, a top plate section closing an upper end of the cap ring section, and an inflow port provided at the top plate section, the inflow port has a liquid guide pipe section projecting downward from the top plate section, the liquid guide pipe section has a tubular shape having a vertical cavity surrounded by a liquid guide pipe side surface and a liquid guide pipe bottom surface, an outlet linked to the vertical cavity is formed in the liquid guide pipe side surface, and, at a deeper wall on the opposite side from the outlet, an inside surface of the liquid guide pipe section at the deeper wall is smoothly connected to the liquid guide pipe bottom surface by a curved surface.

An air trap chamber comprising a housing having a tubular body part having an outflow port at a lower end thereof, and a cap part attached to an upper end of the body part, wherein the cap part has a cap ring section, a top plate section closing an upper end of the cap ring section, and an inflow port provided at the top plate section, the inflow port has a liquid guide pipe section projecting downward from the top plate section, the liquid guide pipe section has a tubular shape having a vertical cavity surrounded by a liquid guide pipe side surface and a liquid guide pipe bottom surface, an outlet linked to the vertical cavity is formed in the liquid guide pipe side surface, and a greatest height of the outlet increases from an inside edge toward an outside edge of the liquid guide pipe side surface.

### INDUSTRIAL APPLICABILITY

The air trap chamber of the present disclosure solves at least one of various problems with air trap chambers having an introduction tube, and is useful in the field of medical treatments.

### DESCRIPTION OF REFERENCE CHARACTERS

- 100, 100A: AIR TRAP CHAMBER
- 101, 101A: BODY PART
- 102, 102A, 202A, 202B: CAP PART
- 103: FILTER
- 104, 104A: HOUSING
- 111: OUTFLOW PORT
- 112: LOWER TUBULAR SECTION
- 113: UPPER TUBULAR SECTION
- 114: STEP OF BODY PART
- 115: INSIDE SURFACE FLANGE PORTION
- 115a: FLANGE SECTION INCLINED SURFACE
- 115b: FLANGE END SURFACE
- 116: LIQUID SURFACE POSITION INDICATOR
- 121, 221: INFLOW PORT
- 121a: TUBE INSERTION SECTION
- 121b: TUBE STOP SECTION
- 122, 222, 223: PORT
- 124, 224: CAP TOP PLATE SECTION
- 125, 225: CAP RING SECTION
- 125a: INCLINED SURFACE
- 125b: LOWER END SURFACE
- 126: CAP FLANGE SECTION
- 141: BLOOD LAYER
- 142: TURNING FLOW
- 151, 251A, 251B: LIQUID GUIDE PIPE SECTION
- 151a: UPPER SECTION
- 151b: LOWER SECTION
- 152: BOTTOM SURFACE
- 153, 283A, 283B: OUTLET
- 154: SIDEWALL PLANE
- 156: INCLINED SURFACE
- 157: GAP
- 165a: CAP UPPER RING SECTION
- 165b: CAP LOWER RING SECTION
- 171: FLOW RECTIFYING WALL
- 271: VERTICAL CAVITY
- 273: UPPER SECTION
- 274: LOWER SECTION
- 281: LIQUID GUIDE PIPE SIDE SURFACE
- 282A, 282B: LIQUID GUIDE PIPE BOTTOM SURFACE
- 285: UPPER SECTION
- 286: MIDDLE SECTION
- 287: LOWER SECTION
- 291: INSIDE SURFACE
- 292, 294: DEEPER WALL
- 293: TRANSITION PORTION
- 296: EJECTOR PIN MARK
- 297: REINFORCING RIB
- 298: EJECTOR PIN MARK
- 302: SLIDE PIN

## Claims

1. An air trap chamber comprising a housing having a tubular body part having an outflow port at a lower end thereof, and a cap part attached to an upper end of the body part, wherein
the cap part has a cap ring section, a top plate section closing an upper end of the cap ring section, and an inflow port provided at the top plate section,
the inflow port has a liquid guide pipe section projecting downward from the top plate section,
the liquid guide pipe section has a tubular shape having a vertical cavity surrounded by a liquid guide pipe side surface and a liquid guide pipe bottom surface, an outlet linked to the vertical cavity is formed in the liquid guide pipe side surface, and
a greatest height connecting a lowermost end and an uppermost end of an opening surface of the outlet is greater than a depth of the vertical cavity between the opening of the vertical cavity and a deeper wall at a height position of an upper end of a boundary between the outlet and the vertical cavity.

2. The air trap chamber of claim 1,
wherein
the vertical cavity has a middle section at which an inside wall surface thereof closer to the outlet is an inclined surface that is inclined toward the deeper wall on the opposite side from the outlet, and an upper section above the middle section, and
an inclination angle of the inclined surface is greater than that of the upper section.

3. The air trap chamber of claim 2,
wherein
the liquid guide pipe bottom surface extends toward the opening surface of the outlet beyond an extension of the inclined surface.

4. The air trap chamber of claim 1,
wherein
a line connecting both side ends of the opening surface of the outlet is tilted radially outward compared to when said line is parallel to a line connecting a central axis of the cap part and a central axis of the liquid guide pipe section.

5. The air trap chamber of claim 1,
wherein
a tunnel-shaped horizontal cavity is formed between the opening surface of the outlet and the vertical cavity.

6. The air trap chamber of claim 1,
wherein
the vertical cavity has a cylindrical upper section, and a lower section at a height position at which the outlet is formed, and
in a horizontal cross-section of the lower section, the vertical cavity has a linear portion or a curved portion having a curvature smaller than that of an inside wall surface of the upper section, the linear or curved portion being lateral to the outlet.

7. The air trap chamber of claim 1,
wherein
at the deeper wall on the opposite side from the outlet, an inside surface of the liquid guide pipe section at the deeper wall is smoothly connected to the liquid guide pipe bottom surface by a curved surface.

8. The air trap chamber of claim 1,
wherein
a lower end surface of the liquid guide pipe section has an ejector pin mark.

9. The air trap chamber of claim 1,
wherein
an inside cavity of the liquid guide pipe section is formed using a first molding pin, and a second molding pin intersecting the first molding pin,
the first molding pin is for forming an upper section of the vertical cavity, and
the second molding pin is for forming a lower section of the vertical cavity and the outlet.

10. The air trap chamber of claim 2,
wherein
the body part has a liquid surface position indicator indicating a target position of a liquid surface, and
a lower end of the outlet is located between an upper end of the body part and the liquid surface position indicator.

11. The air trap chamber of claim 1,
wherein
a stagnation reduction means is provided between the liquid guide pipe section and an inside wall surface of the body part, and
the stagnation reduction means is a gap formed between the liquid guide pipe section and the inside wall surface of the body part, the gap becoming wider downward, or a flow rectifying wall that is formed to project from the liquid guide pipe section toward the inside wall surface of the body part at the deeper wall on the opposite side from the outlet, and is configured to guide the flow of a liquid discharged from the outlet and then flowing along the inside wall surface of the body part, inward along a radial direction of the body part.

12. An air trap chamber comprising a housing having a tubular body part having an outflow port at a lower end thereof, and a cap part attached to an upper end of the body part,
wherein
the cap part has a cap ring section, a top plate section closing an upper end of the cap ring section, and an inflow port provided at the top plate section,
the inflow port has a liquid guide pipe section projecting downward from the top plate section,
the liquid guide pipe section has a tubular shape having a vertical cavity surrounded by a liquid guide pipe side surface and a liquid guide pipe bottom surface, an outlet linked to the vertical cavity is formed in the liquid guide pipe side surface, and
the vertical cavity has a portion at which an inside wall surface thereof closer to the outlet is an inclined surface that is inclined toward a deeper wall on the opposite side from the outlet.

13. An air trap chamber comprising a housing having a tubular body part having an outflow port at a lower end thereof, and a cap part attached to an upper end of the body part,
wherein
the cap part has a cap ring section, a top plate section closing an upper end of the cap ring section, and an inflow port provided at the top plate section,
the inflow port has a liquid guide pipe section integrally molded with the top plate section and projecting downward from the top plate section,
the liquid guide pipe section has a tubular shape surrounded by a liquid guide pipe side surface and a liquid guide pipe bottom surface, and having an outlet in the liquid guide pipe side surface, and
a lower end surface of the liquid guide pipe section has an ejector pin mark.

14. An air trap chamber comprising a housing having a tubular body part having an outflow port at a lower end thereof, and a cap part attached to an upper end of the body part,
wherein
the cap part has a cap ring section, a top plate section closing an upper end of the cap ring section, and an inflow port, and
the inflow port is fitted into the body part.

15. An air trap chamber comprising a housing having a tubular body part having an outflow port at a lower end thereof, and a cap part attached to an upper end of the body part,
wherein
the cap part has a cap ring section, a top plate section closing an upper end of the cap ring section, and an inflow port provided at the top plate section,
the inflow port has a liquid guide pipe section located in the body part, projecting downward from the top plate section, and having an outlet in a side surface thereof,
a stagnation reduction means is provided between the liquid guide pipe section and an inside wall surface of the body part, and
the stagnation reduction means is a gap formed between the liquid guide pipe section and the inside wall surface of the body part, the gap becoming wider downward, or a flow rectifying wall that is formed to project from the liquid guide pipe section toward the inside wall surface of the body part at the deeper wall on the opposite side from the outlet, and is configured to guide the flow of a liquid discharged from the outlet and then flowing along the inside wall surface of the body part, inward along a radial direction of the body part.
